# EUROPEAN PATENT SPECIFICATION

(11) **EP 1 871 872 B1**
(45) Date of publication and mention of the grant of the patent: **04.09.2013**
(21) Application number: 06702937.1
(22) Date of filing: 20.01.2006
(51) Int. Cl.: C12N 5/0783

(54) **METHOD FOR ACTIVATING CD8 T CELLS**
VERFAHREN ZUR AKTIVIERUNG VON CD8-T-ZELLEN
PROCEDE D'ACTIVATION DES LYMPHOCYTES T CD8

(30) Priority: 21.04.2005 KR 20050033191
(43) Date of publication of application: 02.01.2008
(73) Proprietor: Korea University Industry and Academy Cooperation, Seongbuk-gu Seoul 136-701 (KR)
(72) Inventor: PARK, Yong, Keun, Seoul 132-010 (KR); YOON, Won Suck, Nowon-gu Seoul 139-241 (KR)
(74) Representative: Regimbeau
(86) International application number: PCT/KR2006/000228
(87) International publication number: WO 2006/112587

(56) References cited:
- US-A1- 2003 039 628
- US-A1- 2003 224 520
- US-B2- 6 498 006
- CERWENKA; ET AL: "Naive, effector, and memory CD8 T Cells in protection against pulmonary influenza virus infection: homing properties rather than initial frequencies are crucial" THE JOURNAL OF IMMUNOLOGY, vol. 163, no. 10, 1999, pages 5535-5543, XP002498649
- DE BREE GODELIEVE J ET AL: "Selective accumulation of differentiated CD8(+) T cells specific for respiratory viruses in the human lung" J EXP MED,, vol. 202, no. 10, 21 November 2005 (2005-11-21), pages 1433-1442, XP002498650
- MCMICHAEL A: "CYTOTOXIC T LYMPHOCYTES SPECIFIC FOR INFLUENZA VIRUS" CURRENT TOPICS IN MICROBIOLOGY AND IMMUNOLOGY, SPRINGER, BERLIN, DE, vol. 189, 1 January 1994 (1994-01-01), pages 75-92, XP001057618 ISSN: 0070-217X
- LAWRENCE CW ET AL: "Activation, differentiation, and migration of naive virus-specific CD8+ T cells during pulmonary influenza virus infection" THE JOURNAL OF IMUNOLOGY,, vol. 173, no. 2, 1 January 2004 (2004-01-01), pages 1209-1218, XP002498651
- PRICE GRAEME E ET AL: "Perforin and Fas cytolytic pathways coordinately shape the selection and diversity of CD8(+)-T-cell escape variants of influenza virus" JOURNAL OF VIROLOGY,, vol. 79, no. 13, July 2005 (2005-07), pages 8545-8559, XP002498652
- FALCHETTI R ET AL: "Splenic CD4+ and CD8+ T cells from influenza immune mice concurrently produce in vitro IL2, IL4, and IFN-gamma" CELLULAR IMMUNOLOGY,, vol. 170, 1 January 1996 (1996-01-01), pages 222-229, XP002498653
- LAWRENCE C.W.: 'Activation, Differentiation, and Migration of Naive Virus-Specific CD8+ T Cells during Pulmonary Influenza Virus Infection' J. IMMUNOLOGY vol. 173, 15 July 2004, pages 1209 - 1218, XP002498651

## Description

### Technical Field

The present invention relates a method for activating a CD8 T cell which comprises following steps: (1) separating CD8 T cells; and (2) cultivating *in vitro* with a culture broth containing cytokines including all of GM-CSF, IFN-γ, TNF-α, lectin, IL-2 and IL-4; and a therapeutic composition for preventing or treating infectious diseases of virus which comprises the CD8 T cell activated by the method.

### Background Art

*Influenza* virus is a pathogenic and causative agent of bad cold. The virus lives in a somatic cell parasitically and, after the parasitism, distrupts the cell for proliferation pathologically.

Because the virus is parasitic on the somatic cell, drugs showing direct medical effect on the viral infection have not beeen developed. The viral infection has been treated by using an agent relieving the symptom of chill, vomiting or fever which accompanies with the viral infection while expecting a medical effect by the natural immune system.

Recently, a zinc gel spray-type drug was developed which was directly administered to the nasal mucosa. However, the drug induces a medical effect by preventing the influenza virus from attaching to the cell surface rather than directly treating the virus.

In a research field of drugs, immune cells etc. are being investigated to develop a therapeutic agent. For this therapeutic method, the immune cells collected from a particular subject and activated before administered to a patient directly. Presently, immune cells activated by APC cell and specific antibody etc. have been chosen for therapeutic use.

In order to settle above-mentioned problems, the present inventors have found that a CD8 T cell is activated synergistically by adding a culture broth containing GM-CSF, IFN-γ, TNF-α; lectin, IL-2 and IL-4 and further, the resulting CD8 T cell can treat infectious diseases of virus and prevent their re-infections effectively and completed the invention successfully.

### Disclosure Of Invention

The object of the present invention is to provide a method for *in vitro* activating a CD8 T cell that comprises steps: (1) separating CD8 T cells from a biological specimen of subject; and (2) cultivating with a culture broth containing cytokines including all of GM-CSF, IFN-γ, TNF-α, lectin, IL-2 and IL-4.

The other object of the present invention is to provide a therapeutic composition for preventing or treating infectious diseases of virus that comprises the CD8 T cell activated by the method.

Hereinafter, the present invention will be described more clearly as follows.

In one embodiment of the present invention, a method for *in vitro* activating a CD8 T cell that comprises steps: (1) separating CD8 T cells from a biological specimen of subject; and (2) cultivating the CD8 T cells with a culture medium containing cytokines including GM-CSF, IFN-γ, TNF-α, lectin, IL-2 and IL-4 is provided.

The T cell denotes a T cell population that participates in an immune reaction and expresses a specific marker on the cell surface. For a desired use of the present invention, only T cell having a CD8 marker is selected among the T cell population. Preferably, the T cell having a CD8 marker can be cytotoxic T cell (Tc cell) that directly removes virus-infected cells or cancer cells by lysing the cells; and suppressor T cell (Ts cell).

In the description of the present invention, "subject" is a donor providing a mononuclear cell such as T cell and includes all organisms if having a vascular system and hematopoietic cell. Preferably, the subject can be a vertebrate selected among cow, horses, sheep, pigs, goat, camels, antelopes, dogs, mice and the like.

The T cell, that is to say the CD8 T cell can be isolated from various biological samples including mononuclear cell. Preferably, the T cell can be purified among blood, plasma, lymph node, spleen, thymus, bone marrow and the like.

The process for isolating the T cell is not limited. Preferably, the T cell can be isolated by depending upon cell density, affinity of antibody against cell surface epitope, cell size, degree of fluorescent emission. In detail, the T cell is isolated by conducting a density gradient centrifugation using albumin, dextran, Ficoll, metrizamid, Percoll and the like; (MACS) etc. using an antibody; a centrifugal elutriation etc. depending upon cell size; and a FACS using fluorescence.

In the present invention, the T cell is isolated by performing a magnetic activated cell sorter using an anti-CD8 T cell antibody in order to purify a CD8 T cell from a mononuclear cell exclusively.

The CD8 T cell isolated above is cultivated by a conventional method and *in vitro* activated. Preferably, the T cell is cultivated by using a culture medium containing nutrients essential for cell growth and survival. Preferably, the culture medium is comprised of various sources of carbon, nitrogen and trace elements; more preferably, a culture media containing serum; and most preferably, commercial media such as DMEM and RPMI.

In the description of the present invention, "activation of T cell" is to stimulate a T cell to participate in an immune reaction. The T cell can be activated by various signals. Considering the objects of the present invention, the activation of T cell denotes an activation of CD8 T cell.

The present inventors have confirmed that a CD8 T cell is activated synergistically by adding a culture mixture containing GM-CSF, IFN-γ, TNF-α, lectin, IL-2 and IL-4. In detail, experimental mice are observed to have 60% of survival rate, when they are injected with the CD8 T cell activated by cytokines according to the above-mentioned process, in spite of 100% of death rate by the infection of *Influenza* virus A. In contrast, experimental mice injected with the CD4 T cell do not have influence on the death rate even though delaying the time to death. As a consequence, it is noted that the CD8 T cell is very effective to treat infectious diseases caused by virus pertaining to *Orthomyxoviridae* family.

In addition, the experimental mice survived by injecting the CD8 T cell are observed to prevent re-infections of *Influenza* virus. As a consequence, it is noted that the CD8 T cell has an outstanding efficacy as a vaccine.

In the method for *in vitro* activating a CD8 T cell, the concentration of cytokines included in the culture broth are adjusted in the ranges of 0.05 to 0.2 µg/ml of GM-CSF, 0.5 to 2 µg/ml of IFN-γ, 0.05 to 0.2 µg/ml of TNF-α, 40 to 60 µg/ml of lectin, 0.05 to 0.2 µg/ml of IL-2 and 0.05 to 0.2 µg/ml of IL-4.

The cytokine is a wild type of GM-CSF, IFN-γ, TNF-α, lectin, IL-2 or IL-4 derived from various subjects, but can be a fragment or variant of GM-CSF, IFN-γ, TNF-α, lectin or IL-4, if maintaining a biological activity of the wild type.

The culture broth containing the cytokines can include additional ingredients for activating CD8 T cell. Preferably, the additional ingredient can be IL-12, FLT3-ligand, Lipopolysaccharide (LPS) and the like.

Within an effective dose, the process adding the cytokine mixture into a culture broth, the timing, and the number of addition are not particularly limited in order to activate the CD8 T cell. The cytokine mixture can be added while the CD8 T cell is cultivated and otherwise, several times in some interval after cultivated. In addition, within an effective dose, the cytokine mixture can be administered once or multiple times and the timing adding each cytokine can be controlled properly. Preferably, all the cytokines can be treated once while the cell is cultivated. Preferably, the CD8 T cell is further cultivated for 1 to 4 days even after the cytokines are added.

In the present invention, the simple and effective method for stimulating a CD8 T cell activity that is an important factor determining the efficiency of cell therapy without any toxicity is provided. The resulting CD8 T cell activated by the above-mentioned method can be applied to treat various kinds of cancers, virus infections and immune diseases, as a biological medicine for multiple uses.

In another embodiment of the present invention, a therapeutic composition for preventing or treating infectious diseases of virus that comprises the CD8 T cell activated by the above-mentioned method is provided.

In the other embodiment of the present invention, a method for preventing or treating infectious diseases of virus by administering a therapeutically effective amount of the CD8 T cell activated by the above-mentioned method to a patient.

In the description of the present invention, "prevention" designates all behaviors that inhibit a viral infection or delay an invasion by administering the CD8 T cell activated above to a patient.

In the description of the present invention, "treatment" means all behaviors that improve and advantageously modify a symptom of viral infection by administering the CD8 T cell activated above to a patient.

In the description of the present invention, "patient" denotes human and animals including cow, horse, sheep, pig, goat, camel, antelope, dog and the like that can be improved in infectious diseases of virus by administering the CD8 T cell activated above. The infectious diseases of virus can be prevented or treated efficiently by administering the CD8 T cell activated above in a cytokine mixture comprised of GM-CSF, IFN-y, TNF-α, lectin, IL-2 and/or IL-4 to a patient.

In the method of the present invention, the infectious disease that can be treated by administering the CD8 T cell activated above is limited an infectious disease caused by *Influenzavirus* A, *Influenzavirus* B or *Influenzavirus* C.

The CD8 T cell can be administered through any pathway, if possible to reach a target tissue. In detail, the cell can be administered parenterally and for example, it can be utilized by intra-peritoneal injection, intravenous, intra-muscular, subcutaneous or intra-dermal injection, but it is not limited. The cell composition of the present invention can be administered by using any apparatus possible to move active ingredients to a target cell. The cell composition can be additionally comprised of conventional pharmaceutical carriers suitable for cell therapy such as physiological saline solution.

The CD8 T cell of the present invention should be administered in a therapeutically effective amount. In the description of the present invention, "therapeutically effective amount" designates an amount sufficient to treat diseases in a reasonable ratio of benefit/risk suitable for medical therapy. The dosage to be ingested will vary, depending on factors such as severity of disease, age, sex, time of administration, method of administration, ratio of discharge, period of treatment, other drugs and medical factors already disclosed in this art. It is important to administer a minimal amount effective in a maximal extent without any adverse action, considering all the factors. The dosage may be determined by those skilled in this art. As a general guide, it is expected that adult patient would ingest once about 1 mg to 1,000 mg of the CD8 T cell according to the present invention. The individual patient with a particular body weight and life style may readily determine the proper dosage by starting out with the general dosage level set forth above and adjust the dosage as necessary to alleviate the disease.

### Brief Description of the Drawings

The above and other objects, features and other advantages of the present invention will be more clearly understood from the following detailed description taken in conjunction with the accompanying drawings, in which;
**FIG. 1** depicts the process for activating CD8 T cell after separating it from a subject schematically;
**FIG. 2** depicts the comparison of cell efficacies on mice infected by *Influenza* virus;
**FIG. 3** depicts the test of vaccine efficacy on *Influenza* infection in mice survived after administering the activated CD8 T cell;
**FIG. 4** depicts the antigen-antibody reaction against *Influenza* virus in sera collected from mice survived after administering the activated CD8 T cell.

### Best Mode for Carrying Out the Invention

Practical and presently preferred embodiments of the present invention are illustrated as shown in the following Examples. However, it will be appreciated that those skilled in the art, on consideration of this disclosure, may make modifications and improvements within the spirit and scope of the present invention.

### <Example 1 > Preparation of CD8 T cell alleviating Influenza infection

Spleen of mice (BALB/c, SLC Japan) was obtained and sonicated with a cell grinder. The resulting cells suspended in RPMI medium were centrifuged at 1,500 rpm and then, 10 ml of RBC lysing buffer (Sigma, USA) was added to react for 10 minutes at room temperature. After that, the resultant was centrifuged at 1,500 rpm to remove erythrocytes by exchanging 10 ml of RPMI media three times. The mononuclear immune cells obtained above were separated by performing MACS method using anti-CD8 T cell antibodies to obtain CD8 T cells. The resulting CD8 T cells were cultivated for 48 hours with RPMI media containing 10% FBS and cytokines.

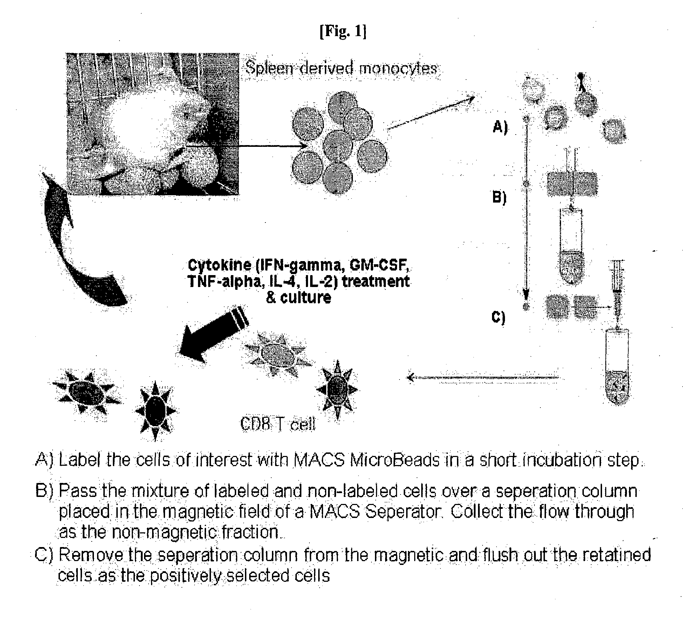

The cytokines added were adjusted in their amounts to GM-CSF 0.1 µg, IFN-y 1, µg, TNF-a 0.1 µg, lectin 50 µg, IL-2 0.1 µg and IL-4 0.1 µg respectively. The cultured cells were collected and then, suspended with PBS buffer before applied for cell therapeutics. FIG. 1 depicts the process for activating the CD8 T cell after separating it from a subject schematically.

### <Example 2> Examination of efficacy of cell therapeutic after Influenza infection in mouse model

In order to induce a viral infection, 1 × 10⁴ of *Influenza* A virus (A/PR/8/34, professor Sung Baek Lin of Yonsei University) were bronchially administered in mice. By the same procedure described in the Example 1, CD8 T cells were activated and suspended with PBS buffer to reach 1 × 10⁶ of cell concentration. Then, the resulting cells were injected intravenously on experimental mice infected by *Influenza* virus.

The survival ratios of mice were measured and compared in the control group injecting only PBS buffer and the experimental group injecting a macrophage cell line or the CD4 T cell (See Table 1).

**<Table 1 >**

| day | control (survived mice/all mice) | macrophage (survived mice/all mice) | CD4 T cell (survived mice/all mice) | CD8 T cell (survived mice/all mice) |
|---|---|---|---|---|
| 2 | 10/10 | 10/10 | 10/10 | 10/10 |
| 4 | 10/10 | 10/10 | 10/10 | 10/10 |
| 8 | 6/10 | 10/10 | 10/10 | 10/10 |
| 10 | 0/10 | 6/10 | 8/10 | 8/10 |
| 12. | 0/10 | 2/10 | 2/10 | 6/10 |
| 14 | 0/10 | 0/10 | 0/10 | 6/10 |
| 16 | 0/10 | 0/10 | 0/10 | 6/10 |

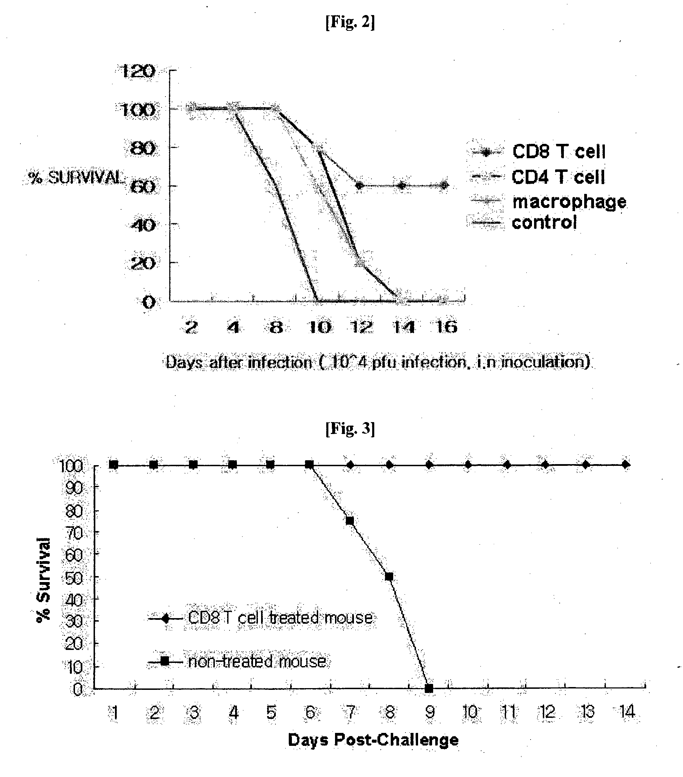

FIG. 2 depicts the comparison of cell efficacies on mice infected by *Influenza.* virus. As a result, it is observed that the CD8 T cell activated above increases a survival ratio of the mice infected by *Influenza* virus after it is administered.

### <Example 3> Examination of vaccine efficacy of cell therapeutic on infectious disease of Influenza in mouse model

Experimental mice were infected by *Influenza* virus and then, treated with the CD8 T cells activated by the same procedure described in the Example 1. 6 mice of the survived group above were administered again with 1 × 10⁴ of *Influenza* virus. In contrast, 6 mice of the control group that has never been infected by *Influenza* virus and injected with only PBS buffer were administered with *Influenza* virus as described above. Survival ratios were compared in the 2 groups to evaluate an efficacy of the vaccine after administering the CD8 T cells.

FIG. 3 depicts the test of vaccine efficacy on *Influenza* infection in mice survived after administering the activated CD8 T cell. As a consequence, it is confirmed that the experimental mice survived above is prevented from the re-infection of *Influenza* virus completely by injecting the CD8 T cell of the present invention.

### <Example 4> Examination of antigen - antibody reaction against Influenza virus

In order to investigate the vaccine efficacy of a cell therapeutic, the experimental mice were treated with the cells and if survived, operated to collect blood from eyeballs. The blood of mice was centrifuged at 3,000 rpm in order to obtain sera. The resulting sera were examined to measure the degree of antibody reaction specific for an antigen of whole *Influenza* A virus by using ELISA method.

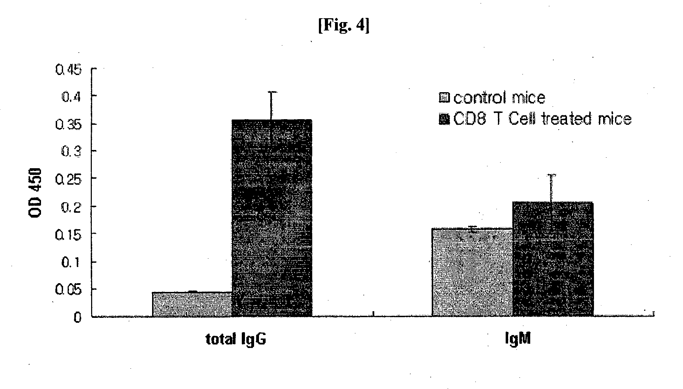

FIG. 4 depicts the antigen-antibody reaction against *Influenza* virus in sera collected from mice survived after administering the activated CD8 T cell. As illustrated in FIG. 4, it is observed that the mice treated with the CD8 T cells indicates the antibody reaction (total IgG) approximately 7-fold sensitive to *Influenza* virus, compared to the normal mice without any treatment. Further, it is examined that the mice treated with the CD8 T cells have a higher IgM value, an immunity index of viscous membrane, compared to the normal mice. Therefore, it is confirmed that the experimental mice treated with the cell therapeutic may stimulate an antibody reaction specific for *Influenza* virus.

## Claims

1. A method for *in vitro* activating a CD8 T cell that comprises steps: (1) separating CD8 T cells from a biological specimen of subject; and (2) cultivating the CD8 T cells with a culture medium containing cytokines including all of GM-CSF, IFN-γ, TNF-α, lectin, IL-2 and IL-4.

2. The method for *in vitro* activating a CD8 T cell according to claim 1, wherein the biological specimen is one selected from a group consisting of blood, plasma, lymph node, spleen, thymus and bone marrow.

3. The method for *in vitro* activating a CD8 T cell according to claim 1, wherein the concentration of cytokines are adjusted in the ranges of 0.05 to 0.2 µg/ml of GM-CSF, 0.5 to 2 µg/ml of IFN-γ, 0.05 to 0.2 µg/ml of TNF-α, 40 to 60 µg/ml of lectin, 0.05 to 0.2 µg/ml of IL-2 and 0.05 to 0.2 µg/ml of IL-4.

4. The method for *in vitro* activating a CD8 T cell according to claim 1, wherein the CD8 T cells are cultivated for 1 to 4 days in Step (2).

5. A therapeutic composition for preventing or treating infectious diseases of virus which comprises the CD8 T cells activated by the method of claim 1, wherein the virus is a *Influenzavirus* A, *Influenzavirus* B or *Influenzavirus* C.

## Patentansprüche

1. Verfahren zum *in vitro*-Aktivieren einer CD8-T-Zelle, welches die folgenden Schritte umfasst: (1) Abtrennen von CD8-T-Zellen aus einer biologischen Probe eines Individuums; und (2) Kultivieren der CD8-T-Zellen mit einem Kulturmedium, welches Zytokine, umfassend alle von GM-CSF, IFN-γ, TNF-α, Lektin, IL-2 und IL-4, enthält.

2. Verfahren zum *in vitro*-Aktivieren einer CD8-T-Zelle nach Anspruch 1, wobei die biologische Probe eine, welche aus einer aus Blut, Plasma, Lymphknoten, Milz, Thymus und Knochenmark bestehenden Gruppe ausgewählt ist, ist.

3. Verfahren zum *in vitro*-Aktivieren einer CD8-T-Zelle nach Anspruch 1, wobei die Konzentration von Zytokinen in den Bereichen von 0,05 bis 0,2 µg/ml GM-CSF, 0,5 bis 2 µg/ml IFN-γ, 0,05 bis 0,2 µg/ml TNF-α, 40 bis 60 µg/ml Lektin, 0,05 bis 0,2 µg/ml IL-2 und 0,05 bis 0,2 µg/ml IL-4 eingestellt wird.

4. Verfahren zum *in vitro*-Aktivieren einer CD8-T-Zelle nach Anspruch 1, wobei die CD8-T-Zellen in Schritt (2) 1 bis 4 Tage kultiviert werden.

5. Therapeutische Zusammensetzung zum Verhüten oder Behandeln von Virusinfektionskrankheiten, welche die durch das Verfahren nach Anspruch 1 aktivierten CD8-T-Zellen umfasst, wobei das Virus ein *Influenza A-Virus, Influenza-B-Virus* oder *Influenza C-Virus* ist.

## Revendications

1. Méthode d'activation *in vitro* d'une cellule T CD8 qui comprend les étapes : (1) de séparation des cellules T CD8 d'un échantillon biologique de sujet ; et (2) de culture des cellules T CD8 avec un milieu de culture contenant des cytokines comprenant la totalité du GM-CSF, de l'IFN-γ, du TNF-α, de la lectine, de l'IL-2 et de l'IL-4.

2. Méthode d'activation *in vitro* d'une cellule T CD8 selon la revendication 1, dans laquelle l'échantillon biologique est un échantillon choisi dans un groupe constitué par du sang, du plasma, des ganglions lymphatiques, la rate, le thymus et la moelle osseuse.

3. Méthode d'activation *in vitro* d'une cellule T CD8 selon la revendication 1, dans laquelle la concentration des cytokines est ajustée dans les plages de 0,05 à 0,2 µg/ml de GM-CSF, 0,5 à 2 µg/ml d'IFN-γ, 0,05 à 0,2 µg/ml de TNF-α, 40 à 60 µg/ml de lectine, 0,05 à 0,2 µg/ml d'IL-2 et 0,05 à 0,2 µg/ml d'IL-4.

4. Méthode d'activation *in vitro* d'une cellule T CD8 selon la revendication 1, dans laquelle les cellules T CD8 sont cultivées pendant 1 à 4 jours dans l'étape (2).

5. Composition thérapeutique destinée à la prévention ou au traitement de maladies infectieuses de virus qui comprend les cellules T CD8 activées par la méthode selon la revendication 1, dans laquelle le virus est un influenzavirus A, influenzavirus B ou un influenzavirus C.
